# EUROPEAN PATENT SPECIFICATION

(11) **EP 4 530 340 B1**
(45) Date of publication and mention of the grant of the patent: **18.03.2026**
(21) Application number: 24386112.7
(22) Date of filing: 17.09.2024
(51) Int. Cl.: C12M 3/00, C12M 1/42

(54) **DYNAMIC COMPRESSION AND FLOW BIOREACTOR FOR THE BIOLOGICAL AND MECHANICAL MATURATION OF CELLS**
DYNAMISCHER KOMPRESSIONS- UND DURCHFLUSSBIOREAKTOR ZUR BIOLOGISCHEN UND MECHANISCHEN REIFUNG VON ZELLEN
BIORÉACTEUR À COMPRESSION ET ÉCOULEMENT DYNAMIQUE POUR LA MATURATION BIOLOGIQUE ET MÉCANIQUE DE CELLULES

(30) Priority: 20.09.2023 GR 20230100757
(43) Date of publication of application: 02.04.2025
(73) Proprietor: BL Nanobiomed Private Company, 570 01 Thessaloniki (GR)
(72) Inventor: Logothetidis, Stergios, 54642 Thessaloniki (GR); Karagkiozaki, Varvara, 54646 Thessaloniki (GR); Tsimenidis, Konstantinos, 55131 Thessaloniki (GR); Orfanos, Alexandros, 56121 Thessaloniki (GR)
(74) Representative: Panagiotidou, Effimia

(56) References cited:
- EP-B1- 2 373 780
- CN-A- 111 893 040
- US-A1- 2014 030 762
- US-A1- 2022 396 753

## Description

The present invention relates to a dynamic compression and flow bioreactor designed for the biological and mechanical maturation of grafts. The invention falls within the field of biotechnology. More specifically, the invention constitutes of a system and a method for applying controlled and circular compression forces to scaffolds and grafts within a chamber of the bioreactor, while simultaneously regulating and controlling the flow of culture media.

The present invention solves the technical problem of producing and preparing grafts on a large scale and within a short timeframe in the medical field, apparent in the current tissue development techniques. A significant problem in regenerative medicine is the need for rapid and efficient cultivation and processing of graft tissues and organs, particularly in urgent medical situations. Despite the progress made in recent years, this process still presents considerable challenges.

The primary technical problem is the maturation time of grafts, where traditional methods of tissue and organ development can be quite time-consuming, depending on the complexity of the graft and the type of tissue. The objective is to create the appropriate controlled environment for graft development, given that traditional methods fall short in creating the necessary biochemical and mechanical conditions needed to ensure that the produced tissues possess the correct mechanical strength, flexibility, and elasticity, which are crucial parameters for their long-term functionality. Moreover, traditional methods are inadequate in providing consistent and reproducible results in graft development.

The artificial creation of conditions similar to human physiology in a bioreactor, is another field with many disadvantages. Solving these technical problems would enable more targeted tissue development, material studies, and the provision of reliable results before these are tested in animals for in-vivo studies (i.e., studies and experiments conducted within a living organism). Resolving this technical problem is imperative because cells and tissues respond entirely differently under static and dynamic conditions (static conditions refer to traditional methods of cell culture in a laboratory flask). The static cell culture methods of the current art do not sufficiently provide the necessary dynamic stimuli that would help bridge the gap between in-vivo and in-vitro models (i.e., studies and experiments conducted outside of a living organism, in a laboratory environment).

The current State of the Art of in-vitro and ex-vivo cellular studies (i.e., studies and experiments conducted on tissues from a living organism but outside the living organism in a laboratory environment) includes the cultivation of cells either by static methods in flasks or in bioreactors that typically aim to create a controlled and dynamic environment that mimics the physiological conditions necessary for tissue development. These bioreactors provide mechanical stimulation, nutrient supply, waste removal, and oxygenation to the cells or tissues being cultured. More specifically, in the existing technology, there are bioreactors that use a continuous flow of nutrient fluid to provide nutrients and oxygen to the cells while removing waste. This technique achieves cell culture on both a small and large scale under static conditions (Patent No. US10934518B2US1). Furthermore, in the existing technology, there are rotating bioreactors that use a rotating cultivation vessel to create a low-shear environment for cell growth. The rotation helps in the uniform distribution of nutrients and oxygen and prevents cell sedimentation. They have been used for the cultivation of various types of tissues (Front. Cell Dev. Biol., 05 March 2019, Sec. Stem Cell Research, Volume 7 - 2019). Additionally, other studies in the literature mention the use of a bioreactor that provides mechanical stimulation to cells in laboratory well plates and incubates them within an incubator to study the biological response of meniscal cells (Front. Bioeng. Biotechnol., 03 September 2021, Sec. Regenerative Medicine, Volume 9 - 2021). Moreover, in another study, Microfluidic bioreactors are used that utilize microscale channels and chambers for cell and tissue culture, allowing the simulation of complex tissue architectures and cell-to-cell interactions. Also, in patent EP2373780B1, a bi-directional continuous perfusion bioreactor for tridemsional culture of mamal tissue substitutes has been demonstrated which is a bioreactor suitable for culture of tissue grafts under conditions of changing pressure and shear. The present invention describes a bioreactor that is not limited to the above features, but additionally induces mechanical dynamic movement of the graft inside the chamber.

Although the results from in-vitro models are valuable tools for the preliminary evaluation of the efficacy or cellular response under various conditions, there is a discrepancy between in-vitro and in-vivo models due to the difficulty in creating representative conditions that prevail in the endogenous environment of the human body. This discrepancy creates the problem of the inability to create reliable grafts.

The disadvantages of the current state of the art focus, on the one hand, on static cell studies, which cannot provide the creation of artificial human physiological conditions, and on the other hand, on bioreactors for cell culture of the existing technique, are based solely on a laboratory scale and do not include all the maturation/incubation parameters, such as, for example, mechanical maturation/incubation and continuous flow of nutrient medium. More specifically, the aforementioned bioreactor systems cannot operate autonomously without the use of an incubator to maintain temperature and carbon dioxide at stable levels. Many bioreactors have been designed for small-scale laboratory experiments, and scaling up for large-scale tissue production can be challenging. Achieving reproducible results on a larger scale is a constant obstacle.

Furthermore, to simulate the human biochemical and mechanical environment, cells must receive both mechanical and biological stimuli. This is a significant drawback of the bioreactor systems in the current state of the art, which either provide continuous flow of nutrient medium or provide mechanical stimuli but do not provide both simultaneously. Additionally, a significant disadvantage of certain systems is the absence of internal heating within the bioreactor chamber, making the use of an external incubator necessary. Consequently, the existing systems present the disadvantage of either lacking an incubator and continuous nutrient medium flow but having mechanical stimulation, or having an incubator and continuous nutrient medium flow but lacking mechanical stimulation. Given that these factors are essential for cell culture, the existing systems exhibit increased complexity and a gap between the results obtained in laboratory experiments and their application.

The present invention relates to a dynamic compression and flow bioreactor designed for the biological and mechanical maturation/incubation of grafts. It enables improved tissue development in grafts and provides maturation capabilities under dynamic conditions for tissue engineering and regenerative medicine applications without the use of an external incubator. Prior to bioreactor use, a three-dimensionally printed sample with specific geometry is created, composed of biomaterials enriched with cells, which constitutes the composite graft. The composite graft is placed in the sample holder in the bioreactor chamber, where the appropriate maturation/incubation conditions are applied for at least 15 to 20 consecutive days. The method is performed under conditions of constant temperature of at least 36.6°C to 37.1°C, CO2 supply of at least 4.9% to 5.1%, continuous nutrient fluid supply to the sample at a rate of at least 0.1 mL/min to 0.5 mL/min, and mechanical compression of the sample with mechanical deformation of at least 0.07 mm to 1 mm and a compression:recovery ratio of at least 70%:30%, using the bioreactor of the present invention.

The bioreactor includes the simultaneous continuous supply of nutrient fluid and the application of external mechanical stresses to specific graft structures. The maturation/incubation of the graft is carried out in the bioreactor chamber by inserting the grafts into the appropriate holders, where continuous controlled supply of nutrient fluid is applied at rates of at least 0.1 mL/min to 0.5 mL/min, under stable temperature conditions of at least 36.6°C to 37.1°C, and CO₂ supply of at least 4.8% to 5.1%, leading to the biological stability of the graft. Additionally, the mechanical maturation/incubation of the graft is achieved by applying cyclically repeated stresses to the graft through mechanical compression with a displacement of at least 0.01 mm to 1 mm, at a frequency of 1Hz, and a compression:recovery ratio between 20:80, 30:70, 40:60, 50:50, 60:40, 70:30, 80:20 %, for at least 15 to 20 days and at least 4 to 8 hours per day.

The present invention has advantages over the current state of the art in the following areas.

It offers a highly accurate and controlled method of applying deformation and mechanical forces to the grafts located within the bioreactor chamber.

Additionally, the deformation rate of the graft can be adjusted, as well as the continuous supply of nutrients to the graft through specific cross-sections at the chamber's inlet, ensuring optimized delivery and enhancing cell viability and graft stability.

Furthermore, the bioreactor system is composed of biocompatible materials, and all parts can be disassembled for cleaning, enabling operation under aseptic conditions.

Another advantage of the present invention is that it offers autonomy in use, without the need for an external maturation/incubation chamber, as it provides temperature regulation.

Summarizing the advantages, this specific bioreactor allows for the creation of simulated human body conditions, offering greater reliability in ex-vivo and in-vitro studies. The aim is to reduce the percentage of in-vivo studies on animals, or even to replace them in the future.

The drawings accompanying the invention briefly illustrate the following:
Figure 1. Bioreactor.
Figure 2. Sectional view of the bioreactor in side view.
Figure 3. Perspective view of the bioreactor. Visual style: interior view projection.
Figure 4. Side view of the bioreactor. Visual style: interior view projection.
Figure 5. Upper section of the bioreactor chamber.
Figure 6. Chamber of the bioreactor.
Figure 7. Supports for the bioreactor chamber.
Figure 8. Parts of the bioreactor chamber.
Figure 9. Parts of the bioreactor chamber lid.
Figure 10. Parts of the bioreactor chamber lid: rectangular flange, glass, rectangular flange, metal welding.
Figure 11. Sleeve with seals and guides.
Figure 12. Horizontal longitudinal section of the sleeve with seals and guides.
Figure 13. Parts of the sleeve in assembly order: metal support for the chamber, sleeve guide sealing ring, metal sleeve guide, friction absorption tube, sleeve sealing tube and application flange.
Figure 14. Left and right sleeves connected to the nutrient fluid separators.
Figure 15. Horizontal longitudinal section in side view, from the left and right sleeves connected to the nutrient fluid supply components.
Figure 16. Placement of the movement restriction base for the specimen, between the sleeves with the nutrient fluid separators.
Figure 17. Nutrient fluid supply component. Visual style: with interior and side view projection.
Figure 18. Side and front view of the base of the nutrient fluid separators.
Figure 19. Nutrient fluid separators and alternative flow components.
Figure 20. All parts of a nutrient fluid separator, in assembly order.
Figure 21. Various views of the movement restriction base for the specimen.

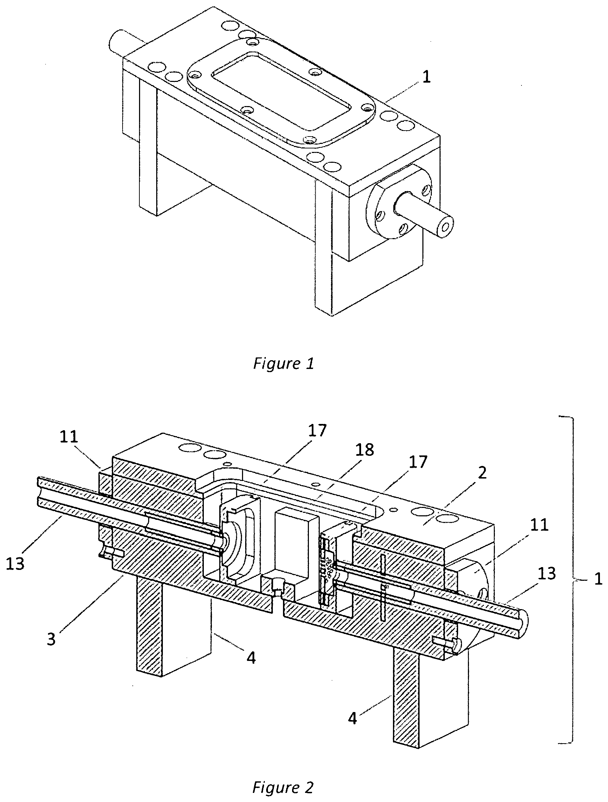

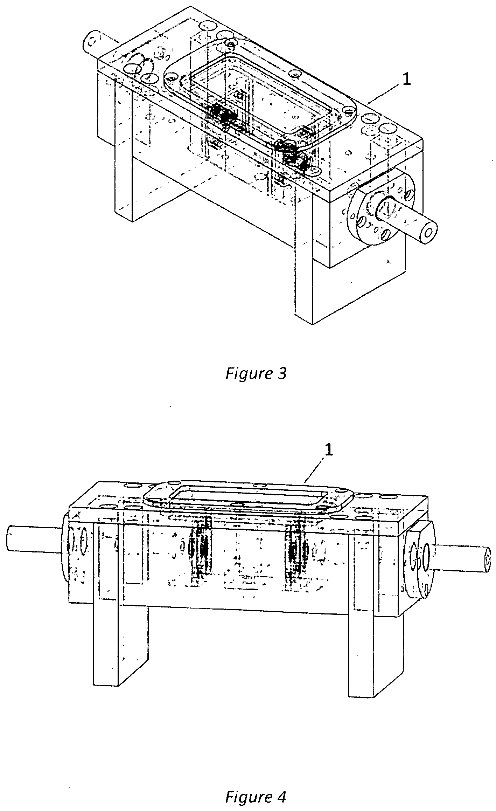

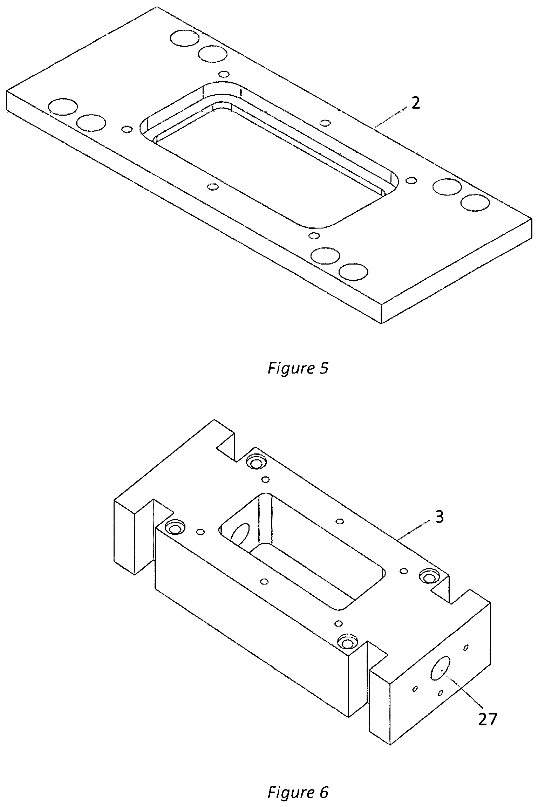

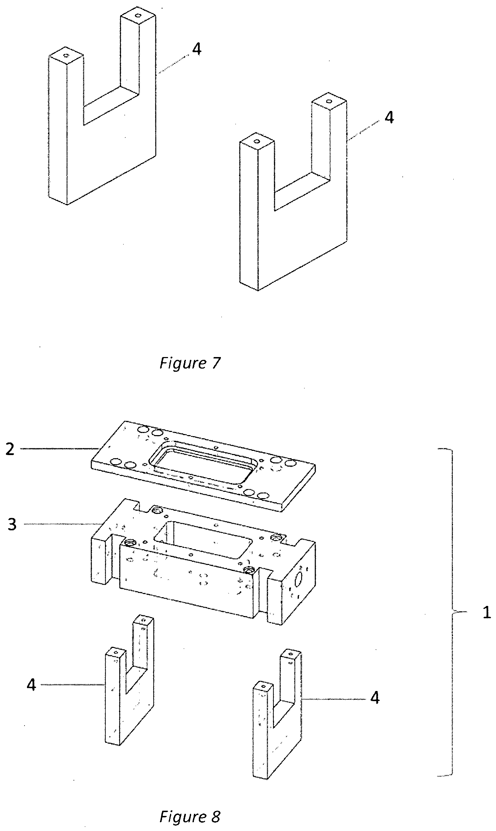

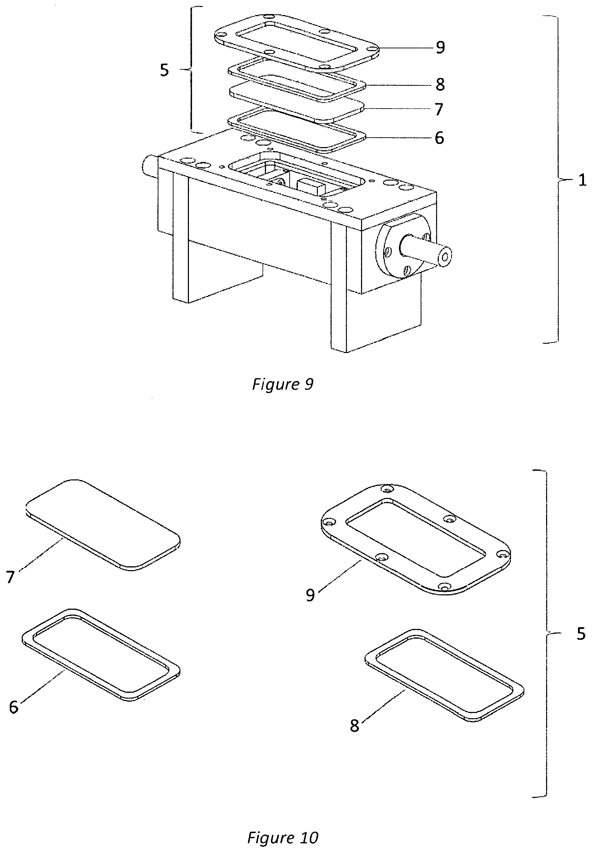

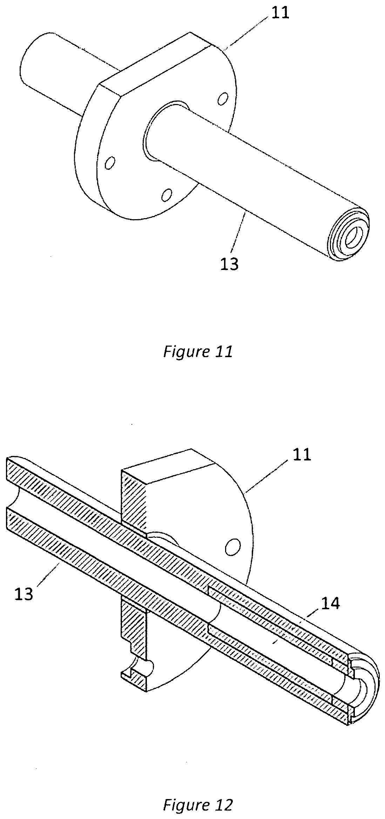

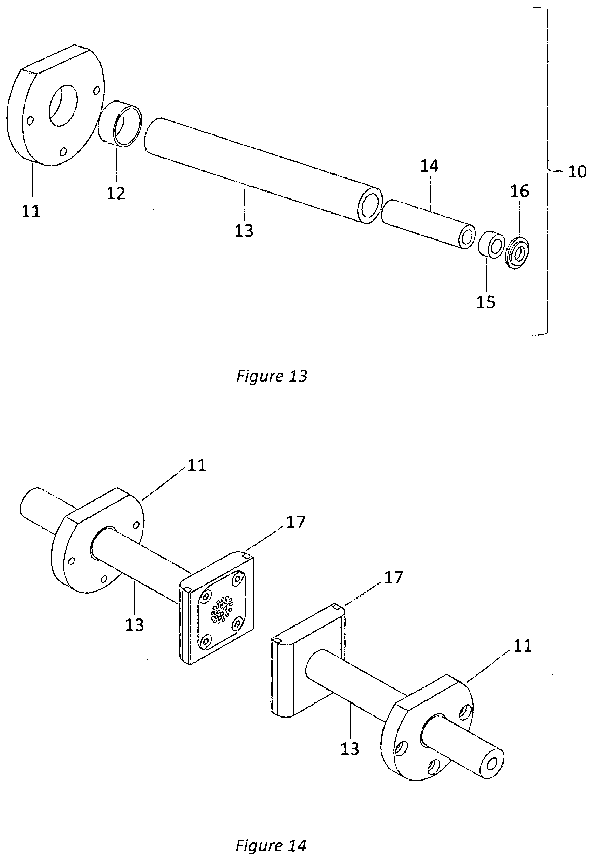

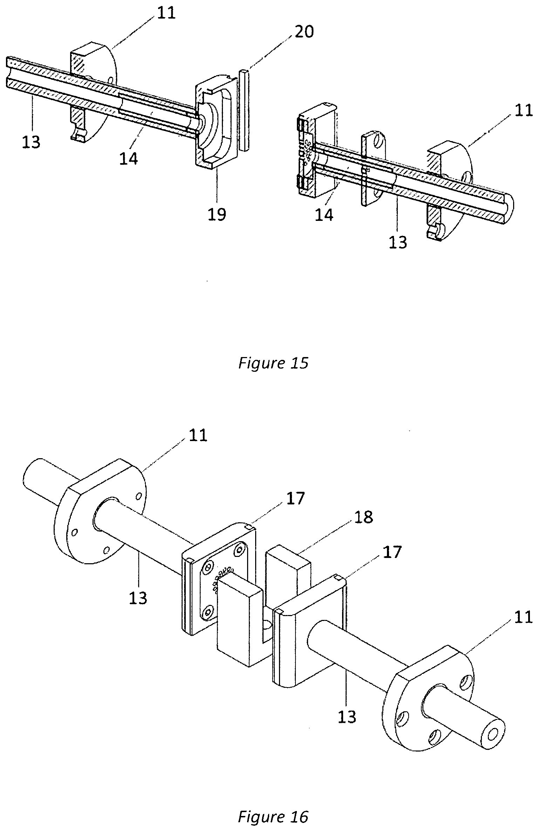

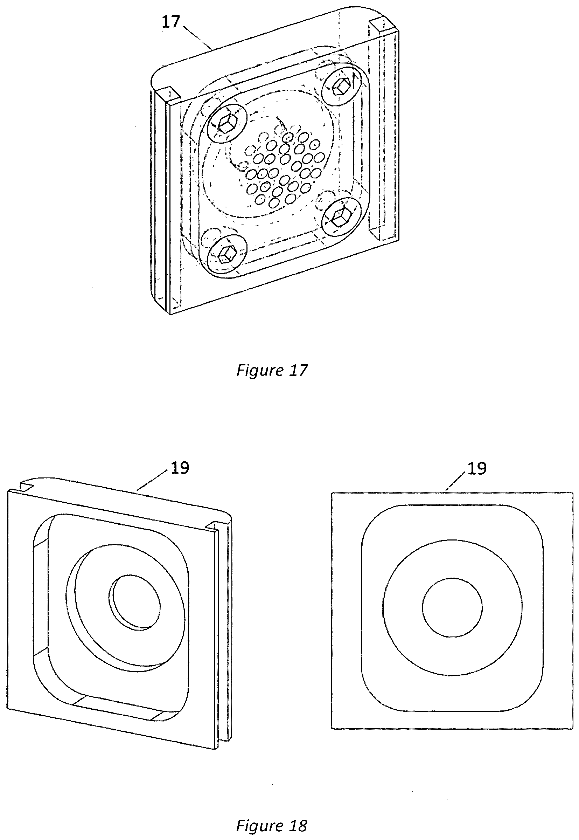

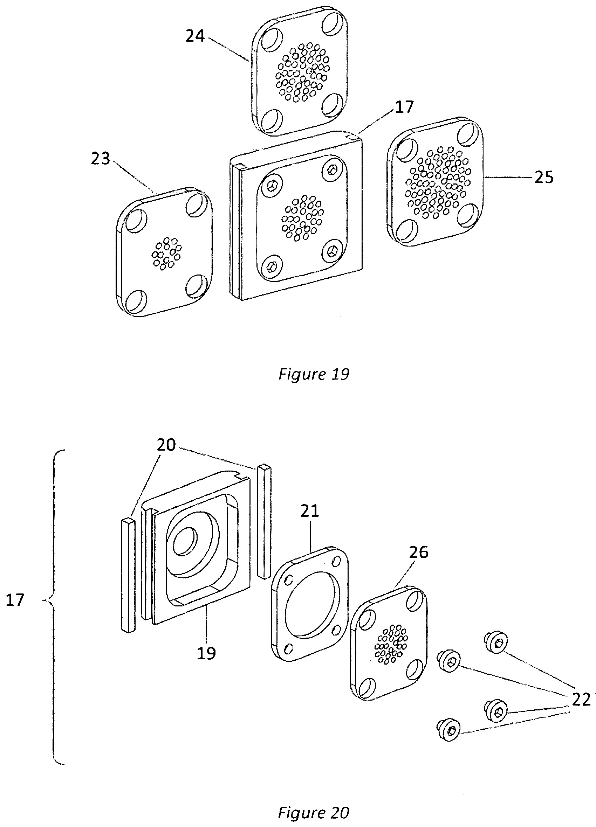

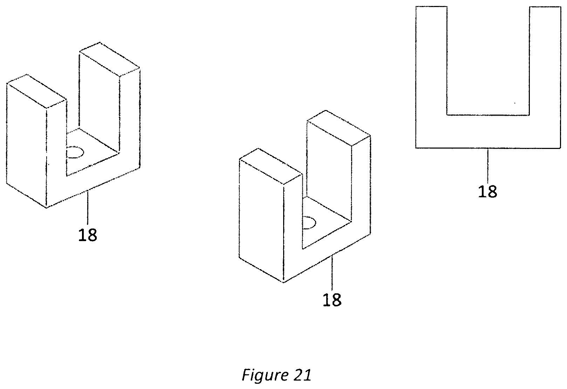

The bioreactor comprises the following elements:
As illustrated in figures 1-10, the bioreactor (1) includes a chamber (3) that is removable from the bioreactor (1). Inside the chamber (3), a sample holder base (18) is placed (figs. 16 and 21), while a metal cover (2) is fitted to the upper part of the chamber (3) (fig. 5), and the chamber (3) is sealed with a sealing cap (5) (figs. 9 and 10). The cap (5) specifically includes a sealing flange with a base (6), a glass cap (7), a sealing and glass protection flange (8), and a metal support cover for the cap (9). Additionally, the removable chamber (3) is secured on chamber supports (4) (figs. 7 and 8). The removable chamber (3) has through openings (27) on its sides, right and left, which serve as recesses for support, into which the sleeves (10) for transferring voltage and nutrient fluid are fitted and enter the interior of the chamber (3). The sleeves (10) (figs. 11, 12, 13, 14, 15, 16) consist of a metal guide (13) inside which a friction absorption tube (14), a sealing tube (15), and an application flange (16) are fitted. The metal guide (13) is attached to a guide sealing ring (12) and then to a metal support (11) on the chamber (3) (fig. 2). At the ends of the sleeves (10), nutrient fluid separators (17) are placed (figs. 2, 11-17). The nutrient fluid separators (17) specifically consist of a metal base (19) (figs. 15, 18, 19, and 20) of the nutrient fluid separators (17), protective parts (20) of the metal base (19), a sealing flange (21), bolts for securing the alternative flow components (22), and alternatively, a nutrient fluid supply component with two rings of holes (23), or a nutrient fluid supply component with four rings of holes (24), or a nutrient fluid supply component with five rings of holes (25), or a nutrient fluid supply component with three rings of holes (26) (fig. 19).

In the embodiment of the inventive concept described herein, the following method is used for creating the composite graft, which will be incubated in the bioreactor, and largely corresponds to the current technique:
The design of the area of interest, which will form the geometry of the composite graft, is carried out, and a file is created in a compatible format to be loaded into a three-dimensional bioprinter. The design file is created on an external computer using any 3D design software. Using the selected software, the object of interest is designed in three-dimensional (3D) form, and an STL format, which is compatible with all three-dimensional printers, is exported. After the above design, the appropriate materials for the printing process, as well as the appropriate cell line, are selected. In this specific case, a bio-ink with the following composition was selected: Gelatin Methacrylate, Sodium Alginate, Calcium Phosphate, Xanthan Gum, Hydroxyapatite, and as a crosslinking agent, the substance LAP (Lithium phenyl-2,4,6-trimethylbenzoylphosphinate). The selected cell line was Human Periodontal Ligament Fibroblasts (HPLF) Stem Cells.

Then, the specific cell line is cultured in a culture flask for at least 3 to 5 days. The culture flask is monitored daily under a microscope, and when its surface reaches more than 90% confluence by the cells, the flask is treated with the enzyme trypsin to detach the cells from the flask. At this stage, centrifugation is performed, and the cells are collected as a pellet in a centrifuge tube. The cells are then resuspended in a nutrient medium.

The printing mixture is prepared at a ratio of 1 part cells to 10 parts bioink. The processes involving cells follow the corresponding protocols for aseptic process conditions. The mixture is transferred to a 3 mL capacity BD Luer Lock extrusion syringe that is compatible with the three-dimensional bioprinter.

Next, the three-dimensional printing of the bioink loaded with cells follows in the selected geometry that has been created from the design file according to Step 1. The three-dimensional bioprinting is carried out by selecting the design file on the bioprinter and inserting the extrusion syringe containing the mixture into the bioprinter head, which is preheated to a temperature of at least 27°C to 32°C, usually for 10 to 15 minutes. Then, printing conditions are applied, which include a printing speed of at least 5 to 25 mm/sec, an extrusion pressure of at least 7 kPa to 35 kPa, and a UV curing speed of at least 1 mm/sec to 8 mm/sec at 405 nm. Additionally, the surface on which the three-dimensional bioprinting is performed is maintained at a temperature of at least 10°C to 25°C. The composite graft is now ready.

In summary, by the above method, the three-dimensionally printed sample is created with the specific geometry of the region of interest, which sample now constitutes the composite graft. Subsequently, the graft is studied in vitro under dynamic conditions in the bioreactor (1).

The creation of the composite graft that will be inserted for maturation/incubation in the bioreactor invention may not be done in the above manner but by other similar synthetic methods of the current art. Additionally, the bioreactor invention operates in the exact same way if a simple graft is introduced, without the prior printing method described in the above steps.

The following is a description of the method of the present invention and the operation of the reactor for implementing the method.

As depicted in Fig. 1, at this stage, the removable chamber (3) of the bioreactor (1) is prepared to receive the graft. The preparation includes disassembling all parts of the chamber (3) of the bioreactor (1) and disinfecting them with a 70% ethanol solution. Then, the parts of the chamber (3) of the bioreactor (1) (Figs. 8, 9, 10) are transferred to the biological safety chamber of the laboratory where UV radiation is applied for at least 20 minutes to ensure the removal of microorganisms and bacteria. The removable chamber (3) of the bioreactor (1) is assembled within the laboratory's biological safety chamber.

During the operation of the bioreactor (1), the following method is applied:
STEP 1: The graft is placed on the sample receiving base (18) (Fig. 16) located inside the removable chamber (3) of the bioreactor (1) (Fig. 6). The removable chamber (3) is sealed airtight with the sealing lid (5), which contains the lid sealing gasket (6), the lid glass (7), the gasket for sealing and protecting the lid glass (8), and the metal cover supporting the lid (9) (Fig. 9).

STEP 2: Subsequently, the removable chamber (3) of the bioreactor (1) is transferred and stabilized on the supports of the chamber (4) of the bioreactor (1) (Fig. 7). The temperature of the bioreactor (1) is adjusted to at least 36.6°C up to 37.1°C through the heating system of the bioreactor (1), and the CO2 supply is adjusted to at least 4.8% up to 5.1% through an external laboratory cylinder.

STEP 3: Continuous and controlled supply of nutrient fluid is applied through the laboratory's peristaltic pump and through the tension and nutrient fluid transfer sleeves (10) (Fig. 12). The flow enters the removable chamber (3) of the bioreactor (1) after passing through the nutrient fluid separators (16) (Fig. 16). The nutrient fluid contains DMEM (Dulbecco's Modified Eagle Medium) enriched with 10% FBS (Fetal Bovine Serum) and 0.5% Penicillin-Streptomycin and is supplied at flow rates of 0.1, 0.2, 0.3, 0.4, 0.5 mL/min, ensuring cell nourishment, waste removal, and oxygenation. STEP 3 ensures the biological maturation/incubation of the graft.

STEP 4: The following dynamic conditions are applied to induce the mechanical maturation/incubation of the graft. This is achieved by applying cyclically repetitive stresses to the graft through mechanical compression from the tension and nutrient fluid transfer sleeves (10). The conditions include dynamic deformation of the graft for mechanical compression from 0.01 to 1 mm, at a constant displacement frequency of 1 Hz, and an adjustable compression:recovery ratio between 20:80, 30:70, 40:60, 50:50, 60:40, 70:30, 80:20%. The application of mechanical stresses to the graft is carried out for at least 4 to 8 hours per day and for at least 15 to 20 consecutive days. All parameters are regulated from the system's LCD screen, which commands the bioreactor (1), and the compression application measurements are recorded in the system's recorder and displayed through a continuous force diagram in grams (g) over time in seconds (sec).

STEP 5: After incubation, the removable chamber (3) of the bioreactor (1) is disassembled from the supports of the chamber (4) and transferred to a biological safety chamber in the laboratory. The sealing lid (5) is removed, and the graft is extracted from the removable chamber (3) of the bioreactor (1) for further processing to evaluate its biological and mechanical response.

Specifically, the graft is transferred to a well plate, and the cell response is determined using MTT (3-(4,5-dimethylthiazol-2-yl)-2,5-diphenyltetrazolium bromide) and simultaneous Live/Dead cell staining using Fluorescein Diacetate (FDA) and Propidium Iodide (PI). The cell response is studied under a light microscope.

The inventive method described above is applied in the same exact way if, instead of bioink consisting of Gelatin Methacrylate, Sodium Alginate, Calcium Phosphate, Xanthan Gum, Hydroxyapatite, and the crosslinking agent LAP (Lithium phenyl-2,4,6-trimethylbenzoylphosphinate), any bioink is used that primarily contains Gelatin Methacrylate, Sodium Alginate, as well as Collagen, Hyaluronic Acid, Fibrin, Chitosan, Polyethylene Glycol (PEG), Polyvinyl Alcohol (PVA), Poly(D,L-lactide-co-glycolide) (PLGA), or Polycaprolactone (PCL).

Similarly, the inventive concept described herein is implemented in exactly the same manner, provided that instead of selecting Human Periodontal Ligament Fibroblasts (HPLF) Stem Cells as the cell line, cells from the categories Adipose Cell System, Cardiac Cell System, Dermal Cell System, Endocrine Cell System, Hematopoietic Cell System, Hepatic Cell System, Lymphatic Cell System, Mammary Cell System, Neural Cell System, Ocular Cell System, Oral Cell System, Skeletal Cell System are chosen, such as, by way of example, Human Bone Marrow Mesenchymal Stem Cells (HMSC-bm), Adipose-Derived Stem Cells (ASCs), Dental Pulp Stem Cells (DPSCs), Synovial Membrane-Derived Mesenchymal Stem Cells (SM-MSCs), Human Oral Keratinocytes (HOK), Human Oral Fibroblasts (HorF), Human Gingival Fibroblasts (HGnF), Human Osteoblasts (HO), Human Tenocytes (HTC), and Human Chondrocytes (HC).

The invention finds application in the fields of medicine, biology, pharmacy, and biotechnology, and the more specific applications include cell and tissue culture for tissue engineering and regenerative medicine, drug development and controlled drug release, such as disease modeling.

In each component referred to above, an indicative number has been assigned, as set forth in the following list:

### COMPONENT LIST

- 1.: Bioreactor (1)
- 2.: Metal cover (2) of the bioreactor chamber
- 3.: Removable chamber (3) of the bioreactor
- 4.: Supports (4) of the bioreactor chamber
- 5.: Sealing cap (5) of the bioreactor chamber
- 6.: Sealing flange (6) of the cap with the base of the bioreactor chamber
- 7.: Glass (7) of the bioreactor chamber cap
- 8.: Sealing and protective flange (8) for the glass of the cap
- 9.: Metal cover (9) supporting the cap of the bioreactor chamber
- 10.: Sleeves (10) for voltage and nutrient fluid transfer
- 11.: Metal support (11) in the chamber
- 12.: Drying ring (12) for sleeve guides
- 13.: Metal guide (13) for the sleeves
- 14.: Absorption tube (14) for sleeve friction
- 15.: Sealing tube (15) type for the sleeves
- 16.: Application flange (16)
- 17.: Nutrient fluid separator (17)
- 18.: Sample receiving base (18)
- 19.: Metal base (19) of the nutrient fluid separator
- 20.: Protective parts (20) of the metal base of the nutrient fluid separator
- 21.: Sealing flange (21) for the nutrient fluid separator
- 22.: Support screws (22) for the alternative flow components
- 23.: Nutrient fluid supply component with two ring holes (23)
- 24.: Nutrient fluid supply component with four ring holes (24)
- 25.: Nutrient fluid supply component with five ring holes (25)
- 26.: Nutrient fluid supply component with three ring holes (26)
- 27.: Through openings (27) of the removable chamber

## Claims

1. A dynamic compression and mechanical maturation bioreactor (1) for composite grafts, **characterized by** the fact that it comprises:
- a chamber (3) that is removable from the bioreactor (1), the chamber having through openings (27) for the insertion of sleeves (10) for stress transfer and nutrient fluid,
- a sample receiving base (18) inside the chamber,
- a metal cover (2) on the upper part of the chamber (3),
- a chamber (3) sealing cap (5) which includes a cap sealing flange with base (6), a cap glass (7), a sealing and protective glass flange (8), and a metal support cover for the cap (9),
- supports (4) for the removable chamber (3),
- sleeves (10) for stress transfer and nutrient fluid, consisting of a metal guide (13) to which is internally fitted a friction absorption tube (14), a sealing tube (15) and an application flange (16), a sealing ring (12) for the metal guide (13), and a metal support (11) in the chamber (3),
nutrient fluid separators (17) located at the ends of the sleeves (10), consisting of a metal base (19), protective parts (20) of the metal base (19), a sealing flange (21), support bolts for the alternative flow components (22), and a nutrient fluid supply component with two rings of holes (23), or a nutrient fluid supply component with four rings of holes (24), or a nutrient fluid supply component with five rings of holes (25), or a nutrient fluid supply component with three rings of holes (26).

2. A method for the mechanical and biological maturation of grafts, carried out by the dynamic compression and mechanical bioreactor of claim 1, **characterized by** the fact that it comprises the following steps:
STEP 1:
The graft is placed in the sample reception base (18) located within the removable chamber (3) of the bioreactor (1), the removable chamber (3) is sealed airtight with the sealing lid (5), which includes the lid sealing flange (6), the lid glass (7), the lid glass sealing and protection flange (8), and the lid's metal support cover (9),
STEP 2:
the removable chamber (3) of the bioreactor (1) is transferred and stabilized in the supports of the bioreactor's chamber (4), the bioreactor's (1) temperature is adjusted to at least 36.6°C to 37.1°C via the bioreactor's heating system, and CO2 supply is set to at least 4.8% to 5.1% via an external laboratory bottle,
STEP 3:
a continuous and controlled supply of nutrient fluid is applied through a peristaltic pump of the laboratory and through the stress and nutrient fluid transfer sheaths (10), the flow enters the removable chamber (3) of the bioreactor (1) after passing through the nutrient fluid separators (16), the nutrient fluid is supplied at flow rates of 0.1, 0.2, 0.3, 0.4, 0.5 mL/min, ensuring cell nourishment, waste removal, and oxygenation,
STEP 4:
cyclically repeating stresses are applied to the graft for its mechanical maturation/incubation through mechanical compression from the stress and nutrient fluid transfer sheaths (10), the conditions include dynamic deformation of the graft for mechanical compression from 0.01 to 1 mm at a constant displacement frequency of 1 Hz and adjustable compression:retraction ratio between 20:80, 30:70, 40:60, 50:50, 60:40, 70:30, 80:20 %, where the application of mechanical stresses to the graft is carried out for at least 4 to 8 hours per day and for at least 15 to 20 consecutive days,
the graft is ready for further processing.

3. A method for the mechanical and biological maturation of grafts according to claim 2, **characterized in that** all execution parameters of the method are controlled by an LCD screen that sends commands to the bioreactor (1), and the compression application measurements are recorded in the system's recorder and depicted through a continuous force diagram in grams (gr) over time in seconds (sec).

## Patentansprüche

1. Ein Bioreaktor (1) zur dynamischen Kompression und mechanischen Reifung für Verbundtransplantate, **dadurch gekennzeichnet, dass** er Folgendes umfasst:
- eine Kammer (3), die aus dem Bioreaktor (1) entnehmbar ist, wobei die Kammer Durchgangsöffnungen (27) zum Einsetzen von Hülsen (10) zur Kraftübertragung und zur Zufuhr von Nährflüssigkeit aufweist,
- eine Probenaufnahmebasis (18) innerhalb der Kammer,
- eine Metallabdeckung (2) am oberen Teil der Kammer (3),
- einen die Kammer (3) abdichtenden Deckel (5), der einen Deckeldichtflansch mit Basis (6), ein Deckelglas (7), einen Dicht- und Schutzglasflansch (8) sowie eine metallische Stützabdeckung (9) für den Deckel aufweist,
- Halterungen (4) für die entnehmbare Kammer (3),
- Hülsen (10) zur Spannungsübertragung und Nährstoffzufuhr, bestehend aus einer Metallführung (13), in die innen eine Reibungsabsorptionsröhre (14), eine Dichtröhre (15) und ein Auftragsflansch (16) eingesetzt sind, einem Dichtungsring (12) für die Metallführung (13) sowie einer Metallstütze (11) innerhalb der Kammer (3), Nährstoffflüssigkeitstrenner (17), die an den Enden der Hülsen (10) angeordnet sind, bestehend aus einer Metallbasis (19), Schutzteilen (20) der Metallbasis (19), einem Dichtflansch (21), Stützbolzen für die alternativen Strömungskomponenten (22) sowie einer Nährstoffzufuhrkomponente mit zwei Lochringen (23), oder einer Nährstoffzufuhrkomponente mit vier Lochringen (24), oder einer Nährstoffzufuhrkomponente mit fünf Lochringen (25), oder einer Nährstoffzufuhrkomponente mit drei Lochringen (26).

2. Verfahren zur mechanischen und biologischen Reifung von Transplantaten, durchgeführt mittels des dynamischen Kompressions- und Mechanik-Bioreaktors nach Anspruch 1, **dadurch gekennzeichnet, dass** es die folgenden Schritte umfasst:
SCHRITT 1:
Das Transplantat wird in der Probenaufnahmebasis (18) innerhalb der entnehmbaren Kammer (3) des Bioreaktors (1) platziert, die entnehmbare Kammer (3) wird luftdicht mit dem Dichtdeckel (5) verschlossen, der den Deckeldichtflansch (6), das Deckelglas (7), den Dicht- und Schutzglasflansch des Deckels (8) sowie die metallische Stützabdeckung des Deckels (9) umfasst,
SCHRITT 2:
die entnehmbare Kammer (3) des Bioreaktors (1) wird in den Halterungen (4) der Bioreaktorkammer transferiert und stabilisiert, die Temperatur des Bioreaktors (1) wird über das Heizungssystem des Bioreaktors auf mindestens 36,6 C bis 37,1 C eingestellt, und die CO₂-Zufuhr wird über eine externe Laborflasche auf mindestens 4,8 % bis 5,1% geregelt,
SCHRITT 3:
eine kontinuierliche und kontrollierte Zufuhr von Nährflüssigkeit wird über eine peristaltische Pumpe des Labors und über die Hülsen (10) zur Spannungs- und Nährstoffübertragung appliziert, wobei der Fluss nach Durchtritt durch die Nährstoffflüssigkeitstrenner (16) in die entnehmbare Kammer (3) des Bioreaktors (1) gelangt, die Nährflüssigkeit mit Flussraten von 0,1, 0,2, 0,3, 0,4, 0,5 mL/min zugeführt wird, wodurch die Ernährung der Zellen, die Entfernung von Abfallstoffen und die Sauerstoffversorgung sichergestellt werden,
SCHRITT 4:
zyklisch wiederkehrende Belastungen werden auf das Transplantat zur mechanischen Reifung/Inkubation durch mechanische Kompression über die Hülsen (10) zur Spannungs- und Nährstoffübertragung appliziert, wobei die Bedingungen eine dynamische Verformung des Transplantats für die mechanische Kompression von 0,01 bis 1 mm bei einer konstanten Verschiebungsfrequenz von 1 Hz sowie ein einstellbares Kompressions-Rückzugs-Verhältnis von 20:80, 30:70, 40:60, 50:50, 60:40, 70:30, 80:20 % umfassen, wobei die
Applikation mechanischer Belastungen auf das Transplantat mindestens 4 bis 8 Stunden pro Tag und für mindestens 15 bis 20 aufeinanderfolgende Tage erfolgt, sodass das Transplantat für die weitere Verarbeitung bereit ist.

3. Verfahren zur mechanischen und biologischen Reifung von Transplantaten nach Anspruch 2, **dadurch gekennzeichnet, dass** alle Ausführungsparameter des Verfahrens über ein LCD-Display gesteuert werden, das Befehle an den Bioreaktor (1) sendet, und dass die Messungen der Kompressionsapplikation im Systemrekorder aufgezeichnet und durch ein kontinuierliches Kraftdiagramm in Gramm (gr) über Zeit in Sekunden (sec) dargestellt werden.

## Revendications

1. Un bioréacteur (1) à compression dynamique et à maturation mécanique pour greffons composites, **caractérisé par le fait qu'**il comprend:
- une chambre (3) amovible du bioréacteur (1), cette chambre comporte des ouvertures traversantes (27) pour insérer des manchons (10) de transfert des contraintes et du milieu nutritif,
- une base de réception d'échantillons (18) à l'intérieur de la chambre,
- un couvercle métallique (2) sur la partie supérieure de la chambre (3),
- un capuchon d'étanchéité (5) de la chambre (3) qui comprend une bride d'étanchéité du capuchon avec une base (6), un verre de capuchon (7), une bride du verre d'étanchéité et de protection (8) et un couvercle métallique de support pour le capuchon (9),
- des supports (4) pour la chambre amovible (3),
- des manchons (10) de transfert des contraintes et du milieu nutritif, composés d'un guide métallique (13) à l'intérieur duquel sont intégrés un tube d'absorption des frottements (14), un tube d'étanchéité (15) et une bride d'application (16), une bague d'étanchéité (12) pour le guide métallique (13), et un support métallique (11) dans la chambre (3),
- des séparateurs (17) de milieu nutritif, situés aux extrémités des manchons (10), composés d'une base métallique (19), des pièces de protection (20) de la base métallique (19), d'une bride d'étanchéité (21), des boulons de support pour les éléments de l'écoulement alternatif (22), et d'un élément d'alimentation en milieu nutritif avec deux anneaux de trous (23), ou d'un élément d'alimentation en milieu nutritif avec quatre anneaux de trous (24), ou d'un élément d'alimentation en milieu nutritif avec cinq anneaux de trous (25), ou d'un élément d'alimentation en milieu nutritif avec trois anneaux de trous (26).

2. Une méthode de maturation mécanique et biologique des greffons, effectuée par le bioréacteur à compression dynamique et mécanique de la revendication 1 et **caractérisée par le fait qu'**elle comprend les étapes suivantes:
ÉTAPE 1:
Le greffon est placé sur la base de réception d'échantillons (18) située dans la chambre amovible (3) du bioréacteur (1), la chambre amovible (3) étant hermétiquement scellée par le couvercle d'étanchéité (5) qui comprend la bride d'étanchéité (6) du couvercle, le verre (7) du couvercle, la bride d'étanchéité et de protection (8) du verre du couvercle, et le support métallique (9) du couvercle,
ÉTAPE 2:
la chambre amovible (3) du bioréacteur (1) est transférée et stabilisée dans les supports de la chambre (4) du bioréacteur, la température du bioréacteur (1) est réglée à au moins 36.6 °C jusqu'à 37.1 °C par le système de chauffage du bioréacteur, et une alimentation en CO2 est fournie à au moins 4.8% jusqu'à 5.1% à partir d'une bouteille de laboratoire externe,
ÉTAPE 3:
un apport continu et contrôlé de fluide nutritif est appliqué par une pompe péristaltique du laboratoire et par les gaines de transfert de contrainte et de fluide nutritif (10), le flux pénètre dans la chambre amovible (3) du bioréacteur (1) après être passé par les séparateurs de fluide nutritif (16), le fluide nutritif est fourni à des débits de 0,1, 0,2, 0,3, 0,4, 0,5 mL/min, assurant la nutrition cellulaire, l'élimination des déchets et l'oxygénation
ÉTAPE 4:
des contraintes cycliquement répétées sont appliquées au greffon pour sa maturation/incubation mécanique grâce à la compression mécanique par les gaines (10) de transfert des contraintes et du milieu nutritif, les conditions impliquent une déformation dynamique du greffon par une compression mécanique de 0.01 à 1 mm à fréquence de déplacement constante de 1 Hz et à rapport compression/rétraction réglable entre 20:80, 30:70, 40:60, 50:50, 60:40, 70:30, 80:20 %, où l'application des contraintes mécaniques au greffon est effectuée pendant au moins 4 à 8 heures par jour pour au moins 15 à 20 jours consécutifs,
le greffon est prêt pour un traitement ultérieur.

3. Une méthode de maturation mécanique et biologique de greffons selon la revendication 2, **caractérisé en ce que** tous les paramètres d'exécution de la méthode sont contrôlés par un écran LCD qui envoie des commandes au bioréacteur (1), et les mesures de l'application de compression sont enregistrées dans la mémoire du système et représentées sur un diagramme de force continue en grammes (gr) en fonction du temps en secondes (sec).
